# FASCICULE DE BREVET EUROPEEN

(11) **EP 2 247 628 B1**
(45) Date de publication et mention de la délivrance du brevet: **26.03.2014**
(21) Numéro de dépôt: 08870748.4
(22) Date de dépôt: 11.12.2008
(51) Int. Cl.: C08F 8/32, C08F 8/14, C04B 24/24, C08F 20/28, C08F 20/58, C08G 81/02, C04B 24/26, A61K 8/00, C11D 1/00, D21H 21/00, C09K 8/00

(54) **AMELIORATION D'UN PROCEDE DE FABRICATION DE POLYMERES PEIGNES PAR AJOUT D'UN ANTIOXYDANT, POLYMERES OBTENUS ET LEURS APPLICATIONS**
VERBESSERTES VERFAHREN ZUR HERSTELLUNG VON KAMMPOLYMEREN DURCH ZUGABE EINES ANTIOXIDANS, RESULTIERENDE POLYMERE UND VERWENDUNGEN DAVON
IMPROVED METHOD FOR PRODUCING COMB POLYMERS BY ADDING AN ANTIOXIDANT, RESULTING POLYMERS AND USES THEREOF

(30) Priorité: 17.01.2008 FR 0800233
(43) Date de publication de la demande: 10.11.2010
(73) Titulaire: COATEX S.A.S., 69730 Genay (FR)
(72) Inventeur: SUAU, Jean-marc, F-69480 Lucenay (FR); PLATEL, David, F-01800 Saint Maurice De Gourdans (FR)
(86) Numéro de dépôt international: PCT/IB2008/003478
(87) Numéro de publication internationale: WO 2009/090471

(56) Documents cités:
- FR-A- 2 900 930
- US-A1- 2002 132 946
- US-B1- 6 433 114
- PRITCHARD: PLASTIC ADDITIVES: AN A-Z REFERENCE, 1998, XP008094530
- IONESCU: "Chemistry and Technology of Polyols for Polyurethanes" 2005, 19000101 , XP008094529 page 146 - page 148

## Description

La présente invention est relative à l'amélioration d'un procédé de synthèse d'un polymère peigne, disposant d'une chaîne principale (méth)acrylique sur laquelle sont fixés des groupements latéraux polyoxyalkylés.

On sait que de tels polymères apportent notamment aux compositions à base de liants hydrauliques, des propriétés intéressantes en termes de fluidité, de maniabilité ou encore de réduction de leur teneur en eau.

Pendant de longues années, l'homme du métier s'est appuyé sur 4 méthodes de préparation de tels polymères. La plus ancienne consiste à réaliser un copolymère de l'anhydride maléique avec un autre monomère hydrophobe en présence d'un solvant organique (comme le toluène ou la méthyl éthyl cétone), sur lequel sont ensuite greffées des chaînes latérales oxyalkylées (voir par exemple le document WO 97/39037). Or, les solvants organiques demeurent des substances dangereuses qu'il est difficile d'éliminer.

La deuxième voie de synthèse consiste à copolymériser en milieux solvant ou aqueux un monomère (méth)acrylique et un macromonomère du type polyéther (méth)acrylique (voir les documents US 2001 001797 et FR 2 861 399). Le prix élevé du macromonomère de départ (méth)acrylique constitue ici un frein à la mise en oeuvre d'une telle méthode.

Une troisième voie de synthèse consiste à copolymériser en milieux solvant ou aqueux un macromonomère vinylique disposant de chaînes pendantes oxyalkylées avec un monomère (méth)acrylique (voir le document US 2004/235687). Comme précédemment, le prix du macromonomère initial constitue un obstacle pour l'homme du métier.

Il existe une quatrième voie de synthèse qui consiste à estérifier un homo- ou copolymère de l'acide (méth)acrylique par un alcoxy polyoxyalkylène glycol en milieu solvant (voir le document EP 1 016 638) ou aqueux (voir le document FR 2 776 285). Néanmoins, cette méthode ne conduit pas à des rendements satisfaisants.

Aussi, il a été mis au point selon le document FR 2 900 930 une nouvelle méthode de fabrication de ces polymères peignes, palliant aux inconvénients des techniques de synthèse mettant en oeuvre des solvants dangereux ou des macromonomères coûteux, et conduisant à des rendements plus élevés que ceux obtenus par estérification en milieux aqueux.

L'originalité de cette méthode consiste à effectuer la réaction d'estérification sur un homo- ou un copolymère de l'acide (méth)acrylique à l'état sec. Concrètement, on introduit l'homo- ou le copolymère de l'acide (méth)acrylique sous forme sèche dans le milieu fondu contenant les groupements servant à la fonctionnalisation : la réaction de synthèse s'effectue à une température supérieure à 100°C. On diminue ainsi largement le temps de réaction (conduisant à un taux de transformation donné) par rapport à la même synthèse réalisée en milieu aqueux.

Dans le document FR 2 900 930, le taux de transformation est mesuré par indice d'acide selon une méthode de titration des fonctions carboxyliques : les fonctions carboxyliques dosées sont les fonctions qui n'ont pas réagi. Ce document élargit même le concept du greffage sur un polymère sec, à des réactions d'amidification avec un alcoxy polyoxyalkylène amine et d'éthoxylation avec un oxyde d'alkylène.

Sur la base de l'invention réalisée dans le document FR 2 900 930, la Demanderesse a dosé par GPC la quantité de groupements méthoxy n'ayant pas réagi, dans le cas de l'estérification d'un acide polyméthacrylique par du méthoxy polyéthylène glycol. Comme le démontrent les essais n° 1 et 2 de la présente Demande, la valeur obtenue est élevée puisqu'égale à 20 % en poids du méthoxy polyéthylène glycol de départ. Or, la méthode de titration des groupements carboxyliques révèle une disparition totale des fonctions acides : ceci démontre que la réaction d'estérification a été totale.

Cherchant à expliquer de tels résultats qui apparaissent contradictoires, la Demanderesse a su identifier un problème technique inconnu jusqu'alors dans un tel procédé : la présence de réactions secondaires, vraisemblablement catalysées par la température élevée de réaction, et qui conduisent à une libération des chaînes méthoxy pendantes. En vue de pallier à un tel inconvénient, elle est ensuite parvenue à mettre au point un procédé d'amélioration de la technique proposée par l'art antérieur.

Cette amélioration consiste à mettre en oeuvre au moins un antioxydant, avant et/ou pendant l'étape de fonctionnalisation, qui permet de réduire la quantité des fonctions qui n'ont pas réagi, telle que déterminée par GPC. On est ainsi parvenu à limiter de manière très significative le nombre des réactions oxydatives secondaires, qui conduisaient à une libération des chaînes pendantes : le rendement de la réaction s'en trouve largement amélioré. Par antioxydant, on entend une substance possédant la capacité de limiter le clivage des molécules et/ou la propagation des radicaux libres.

Outre l'effet technique très marqué lié à la mise en oeuvre de cet antioxydant, le caractère inventif de la présente invention repose aussi sur la mise à jour d'un problème technique encore inconnu dans un procédé déjà connu : celui de la libération des chaînes pendantes du polymère peigne sous l'effet de la température. Résolvant ensuite ce problème, la Demanderesse est parvenue à améliorer un procédé fondamental de l'art antérieur, puisque permettant à l'homme du métier de s'affranchir de techniques dangereuses (solvants) et / ou coûteuses (macromonomère de départ). Possibilité est donc offerte à l'homme du métier, de produire à moindre coût, sans solvant, de manière rapide et avec un rendement élevé, des polymères peignes disposant d'une chaîne principale (méth)acrylique sur laquelle sont fixés des groupements latéraux polyoxyalkylés.

Aussi, un premier objet de l'invention consiste en un procédé de fabrication de polymères peignes disposant d'une chaîne principale (méth)acrylique et de groupements latéraux polyoxyalkylés, consistant à :
a) réaliser une solution contenant au moins un homopolymère et / ou copolymère de l'acide (méth)acrylique avec au moins un autre monomère,
b) sécher la solution obtenue à l'étape a), en vue d'obtenir un homopolymère et / ou un copolymère de l'acide (méth)acrylique avec au moins un autre monomère à l'état sec,
c) mélanger le produit à l'état sec obtenu selon l'étape b) avec au moins un alcoxy polyoxyalkylène glycol à l'état fondu et / ou au moins un alcoxy polyoxyalkylène amine à l'état fondu et / ou au moins un oxyde d'alkylène à l'état liquide ou gazeux,
d) fonctionnaliser alors l'homopolymère et / ou le copolymère de l'acide (méth)acrylique par :
   - estérification avec au moins un alcoxy polyoxyalkylène glycol,
   - et / ou amidification avec au moins un alcoxy polyoxyalkylène amine,
   - ou éthoxylation avec au moins un oxyde d'alkylène.
et caractérisé en ce qu'on introduit un antioxydant pendant l'étape a) et /ou l'étape b) et / ou l'étape c).

Selon une première variante préférentielle de l'invention, l'antioxydant est une amine ayant au moins un groupement aromatique substitué par une chaîne alkyle, préférentiellement deux groupements aromatiques dont l'un au moins est substitué par une chaîne alkyle ayant de 3 à 9 atomes de carbone et est très préférentiellement le composé dont le numéro CAS est le 68411-46-1. L'Irganox™ 5057 commercialisé par la société CIBA™ est un exemple commercial d'un tel composé.

Selon une deuxième variante préférentielle de l'invention, l'antioxydant est composé organophosphate, préférentiellement un phosphite aromatique et/ou aliphatique, et est très préférentiellement choisi parmi les composés dont les numéros CAS sont 25550-98-5 et 101-02-0. Le Doverphos™ 7 et le Doverphos™ 10 commercialisés par la société DOVER™ sont des exemples commerciaux de tels composés.

La Demanderesse souligne que le caractère inventif de ces 2 variantes préférentielles est notamment supporté par le fait que l'Irganox™ 5057 et les Doverphos™ 7 et 10 n'étaient utilisés jusqu'alors que dans des domaines techniques très éloignés, respectivement pour stabiliser des mousses polyuréthannes et des polyesters.

Le procédé selon l'invention est aussi caractérisé en ce que l'étape c) est effectuée à une température comprise entre 150°C et 250°C, préférentiellement entre 180°C et 220°C.

Le procédé selon l'invention est aussi caractérisé en ce que l'étape c) est effectuée en présence d'un catalyseur, ledit catalyseur étant préférentiellement choisi parmi l'acide p-toluène sulfonique et l'hydroxyde de lithium.

Le procédé selon l'invention est aussi caractérisé en ce que l'alcoxy polyoxyalkylène glycol est un méthoxy polyoxyalkylène glycol, et préférentiellement un méthoxy polyoxyéthylène glycol.

Le procédé selon l'invention est aussi caractérisé en ce que l'alcoxy polyoxyalkylène amine est un méthoxy polyoxyalkylène amine, et préférentiellement un méthoxy polyoxyéthylène amine.

Le procédé selon l'invention est aussi caractérisé en ce que l'oxyde d'alkylène est un oxyde d'éthylène, ou de propylène ou leurs mélanges.

Le procédé selon l'invention est aussi caractérisé en ce que l'homopolymère et / ou le copolymère de l'acide (méth)acrylique est obtenu par des procédés de polymérisation radicalaire en solution, en émulsion directe, en présence de systèmes catalytiques et d'agents de transfert, ou encore par des procédés de polymérisation radicalaire contrôlée et préférentiellement par la polymérisation contrôlée par des nitroxydes (NMP) ou par des cobaltoximes, la polymérisation par transfert d'atome radicalaire (ATRP), la polymérisation radicalaire contrôlée par des dérivés soufrés, choisis parmi des carbamates, des dithioesters ou des trithiocarbonates (RAFT) ou des xanthates.

Le procédé selon l'invention est aussi caractérisé en ce que l'autre monomère du copolymère de l'acide (méth)acrylique est choisi parmi :
- au moins un monomère anionique à insaturation éthylénique et à fonction monocarboxylique qui est préférentiellement l'acide acrylique ou méthacrylique ou leurs mélanges,
- au moins un monomère à insaturation éthylénique, choisi parmi au moins un monomère à insaturation éthylénique et fonction dicarboxylique et est préférentiellement choisi parmi l'acide crotonique, itaconique, maléique, ou encore les anhydrides d'acides carboxyliques, et est préférentiellement l'anhydride maléique ou choisi parmi les monomères à insaturation éthylénique et à fonction sulfonique et est préférentiellement choisi parmi l'acide 2-acrylamido-2-méthyl-propane-sulfonique, l'acide vinyl sulfonique, ou les sels de l'acide allyl éther sulfonate, l'acide styrène sulfonique ou bien encore choisi parmi les monomères à insaturation éthylénique et à fonction phosphorique et est préférentiellement choisi parmi l'acide vinyl phosphorique, le phosphate de méthacrylate d'éthylène glycol, le phosphate de méthacrylate de propylène glycol, le phosphate d'acrylate d'éthylène glycol, le phosphate d'acrylate de propylène glycol et leurs éthoxylats ou bien encore choisi parmi les monomères à insaturation éthylénique et à fonction phosphonique et est préférentiellement l'acide vinyl phosphonique, ou encore choisi parmi la vinylcaprolactone ou la vinylpyrrolidone, ou leurs mélanges,
- au moins un monomère non hydrosoluble tel que les acrylates ou les méthacrylates d'alkyles, ou leurs mélanges,
ou les mélanges de ces monomères.

Le procédé selon l'invention est aussi caractérisé en ce que le séchage de l'homopolymère et / ou du copolymère de l'acide (méth)acrylique réalisé selon l'étape b) est effectué dans un sécheur par atomisation. Toutefois, l'homme du métier saura recourir à toutes les techniques de séchage bien connues de lui en vue de sécher l'homopolymère et / ou le copolymère au cours de l'étape b).

Le procédé selon l'invention est aussi caractérisé en ce que le séchage de l'homopolymère et / ou du copolymère de l'acide (méth)acrylique réalisé selon l'étape b), est effectué jusqu'à obtenir un taux de matière sèche supérieur à 80 %, préférentiellement 90 %, et très préférentiellement supérieur à 95 % du poids dudit homopolymère et / ou dudit copolymère, tel que mesuré par une balance-dessicateur commercialisée par la société METTLER-TOLEDO™ sous le nom de HR83, la mesure étant réalisée par séchage à 150°C jusqu'à obtenir une perte en masse inférieure à 1 mg pendant 30 secondes.

Le procédé selon l'invention est aussi caractérisé en ce qu'il comporte une étape éventuelle e), ultérieure à l'étape d), qui consiste à mettre en solution aqueuse le produit obtenu à l'issu de l'étape d), puis éventuellement à neutraliser totalement ou partiellement ledit produit par un ou plusieurs agents de neutralisation.

Le procédé selon l'invention est aussi caractérisé en ce que les agents de neutralisation sont choisis parmi ceux disposant d'une fonction neutralisante monovalente et sont alors préférentiellement choisis parmi les cations alcalins, et très préférentiellement choisis parmi le sodium, le potassium, le lithium, l'ammonium ou sont préférentiellement choisis parmi les amines primaires, secondaires, tertiaires aliphatiques et / ou cycliques, et très préférentiellement choisis parmi la stéarylamine, les éthanolamines (mono-, di-, triéthanolamine), la mono et diéthylamine, la cyclohexylamine, la méthylcyclohexylamine, l'amino méthyl propanol, la morpholine, ou sont choisis parmi les agents de neutralisation disposant d'une fonction neutralisante divalente et sont préférentiellement choisis parmi les cations divalents alcalino-terreux, et sont très préférentiellement choisis parmi le magnésium, le calcium, le zinc, ou sont choisis parmi les agents de neutralisation disposant d'une fonction neutralisante trivalente et consistent préférentiellement en l'aluminium, ou sont choisis parmi les agents de neutralisation disposant d'une fonction neutralisante plus élevée que la fonction trivalente, ou sont choisis parmi leurs mélanges.

Un deuxième objet de l'invention réside dans les polymères peignes, à l'état sec, disposant d'une chaîne principale (méth)acrylique et de groupements latéraux polyoxyalkylés greffés, caractérisés en ce qu'ils présentent un taux de greffage supérieur à 70 %, préférentiellement 80 %, très préférentiellement 90 %, tel que mesuré par GPC. Bien évidemment, ces polymères sont ceux obtenus par le procédé précédemment décrit (sans mettre naturellement en oeuvre l'étape optionnelle e) de mise en solution aqueuse).

Ces polymères peigne, à l'état sec, sont aussi caractérisés en ce qu'ils présentent un taux de matière sèche supérieur à 80 %, préférentiellement supérieur à 85 %, très préférentiellement supérieur à 90 %, et extrêmement préférentiellement supérieur à 95 % de leur poids, tel que mesuré par une balance-dessicateur commercialisée par la société METTLER-TOLEDO™ sous le nom de HR83, la mesure étant réalisée par séchage à 150°C jusqu'à obtenir une perte en masse inférieure à 1 mg pendant 30 secondes.

Un troisième objet de l'invention réside dans les polymères peignes, en solution aqueuse, disposant d'une chaîne principale (méth)acrylique et de groupements latéraux polyoxyalkylés greffés, caractérisés en ce qu'ils présentent un taux de greffage supérieur à 70 %, préférentiellement 80 %, très préférentiellement 90 %, tel que mesuré par GPC. Bien évidemment, ces polymères sont ceux obtenus par le procédé précédemment décrit et par mise en oeuvre de l'étape e) de mise en solution aqueuse.

Un quatrième objet de la présente invention réside dans les formulations contenant des matières minérales ou organiques, caractérisées en ce qu'elles contiennent au moins un polymère peigne de la présente invention.

Ces formulations sont aussi caractérisées en ce qu'elles sont des formulations aqueuses contenant des matières minérales ou organiques, lesdites formulations étant choisies parmi des dispersions ou des suspensions aqueuses de matières minérales ou organiques, contenant éventuellement un agent de dispersion et / ou un agent d'aide au broyage et / ou un agent anti sédimentation et / ou un agent épaississant, ou des compositions aqueuses à base de liants hydrauliques, et sont préférentiellement des bétons, des mortiers, des laitiers, des coulis, des plâtres, ou des sauce de couchage papetière, ou des peintures, ou des formulations cosmétiques ou détergentes, ou des formulations textiles, ou des formulations céramiques, ou des boues de forage.

Les exemples suivants permettront de mieux appréhender le contenu de l'invention, sans toutefois en limiter la portée.

### EXEMPLES

Dans l'ensemble des exemples de la présente Demande le taux d'estérification est calculé à partir du suivi de l'indice d'acide ce qui permet de déterminer la proportion de fonctions acides n'ayant pas réagi (selon la méthode décrite dans le document FR 2 900 930).

Le poids moléculaire du polymère peigne fabriqué ainsi que la teneur en méthoxy polyalkylène glycol non greffé sont déterminés par GPC selon la méthode suivante.

Le système chromatographique est composé de passeur d'échantillons Waters™ 717, de dégazeur en ligne ERC 3112, d'une pompe HPLC Waters 515, d'un four à colonnes, d'un réfractomètre Waters™ 2410, d'un détecteur viscosimétrique et à diffusion de lumière Viscotek™ T60 A, d'une colonne de garde Ultrahydrogel Waters™, d'une colonne Waters™ Ultrahydrogel linéaire + 2 colonnes Waters Ultrahydrogel 120 (longueur 30 cm ; diamètre 7,8 mm) et d'un système informatique avec logiciel TriSEC 3.0. La phase mobile est préparée à partir d'une solution mère de sulfate de sodium à 666 mM filtrée à 0,1 µm. 10% de cette solution sont mélangés à 5 % d'ACN qsp eau ultra pure, neutralisé à pH 9 avec la soude. Le débit est de 0,8 ml/min, la température des colonnes et du réfractomètre est de 30°C, la sensibilité et le scale facteur du réfractomètre étant égaux à respectivement 4 et 20. Les échantillons possèdent 4 mg sec de produit /ml de phase mobile. Ils sont filtrés à 0,2 µm avant injection. Le volume d'injection est de 100 µl, le temps d'analyse de 50 min. L'étalonnage est réalisé avec le PEO 21k étalon Viscotek™ avec correction de débit sur le pic négatif de l'eau et dextran T70K.

### Exemple 1

Cet exemple illustre le procédé de l'art antérieur tel que décrit dans le document FR 2 900 930 et qui ne met pas en oeuvre d'antioxydant.

On commence par prendre une solution d'un homopolymère de l'acide méthacrylique en solution dans l'eau à 30 % massique, et commercialisé par la société COATEX™ sous le nom TP 941.
Cette solution est ensuite séchée. Elle est introduite dans une chambre de pré-chauffage à une température de 85°C. Le produit est injecté dans la chambre d'atomisation d'un sécheur-atomiseur à travers une buse, sous une pression de 60 bars. On injecte également dans la chambre d'atomisation de l'air chaud à 300°C, ce qui provoque l'évaporation flash de l'eau de la solution de polymère.
Le rendement de ce séchage est supérieur à 99 %, c'est-à-dire que la masse de polymère sec obtenu après séchage est au moins égale à 99 % de la masse de polymère contenu dans la solution aqueuse de départ.
La température du polymère en sortie est égale à 50°C.
Cette opération de séchage est extrêmement rapide, puisqu'elle ne dure que quelques secondes. On récupère alors le polymère sous forme d'une poudre sèche.

### Essai n° 1

Ensuite, dans un réacteur en verre de 0,25 litre muni d'une agitation mécanique et d'un chauffage électrique on mélange :
- 80 g de méthoxy polyéthylène glycol de masse moléculaire égale à 2 000 g/mole, commercialisé par la société CLARIANT™ sous le nom de Polyglykol™ M 2000, sous forme fondu (soit 0,04 mole),
- 0,24 g d'hydroxyde de lithium,
- 13,8 g de l'homopolymère de l'acide méthacrylique sec obtenu par la méthode de séchage précédemment décrite (soit 0,16 mole d'acide méthacrylique).

La réaction d'estérification de l'homopolymère de l'acide méthacrylique par le méthoxy polyéthylène glycol a alors lieu. L'ensemble est dégazé par un courant d'azote pendant 20 minutes. Sous agitation, on monte ensuite progressivement la température jusqu'à 190°C et on met l'ensemble sous vide (20 mm de mercure) cette montée en température s'effectuant en 60 minutes. On cuit alors le mélange durant 120 minutes à 190°C. L'ensemble est alors refroidi et dilué avec 78,3 g d'eau puis neutralisé avec de la soude 50 % jusqu'à pH 8,4.

Avant estérification, l'indice d'acide (IAᵢ) du mélange est égal à 83 mg de potasse par gramme de produit. Après estérification, cet indice (IA_{f}) est égal à 34 mg de potasse par gramme de produit. Ceci se traduit par une consommation totale des fonctions alcools introduites. On obtient un poids moléculaire de 155 600 g/mole. La teneur en méthoxy polyéthylène glycol non réagi est égale à 20 % en poids du méthoxy initial, soit un rendement calculé de méthoxy effectivement greffé de 57 % en poids du méthoxy initial.

### Essai n° 2

Ensuite, dans un réacteur en verre de 0,25 litre muni d'une agitation mécanique et d'un chauffage électrique on mélange :
- 80 g de Polyglykol™ M 2000, sous forme fondu (soit 0,04 mole),
- 1,9 g d'acide p-toluène sulfonique,
- 13,8 g de l'homopolymère de l'acide méthacrylique sec obtenu par la méthode de séchage précédemment décrite (soit 0,16 mole d'acide méthacrylique).

La réaction d'estérification de l'homopolymère de l'acide méthacrylique par le méthoxy polyéthylène glycol a alors lieu. L'ensemble est dégazé par un courant d'azote pendant 20 minutes. Sous agitation, on monte ensuite progressivement la température jusqu'à 190°C et on met l'ensemble sous vide (20 mm de mercure) cette montée en température s'effectuant en 60 minutes. On cuit alors le mélange durant 120 minutes à 190°C. L'ensemble est alors refroidi et dilué avec 78,3 g d'eau puis neutralisé avec de la soude 50 % jusqu'à pH 8,4

Avant estérification, l'indice d'acide du mélange est égal à 83 mg de potasse par gramme de produit. Après estérification, il est égal à 35 mg de potasse par gramme de produit. Ceci se traduit par une consommation totale des fonctions alcools introduites. On obtient un poids moléculaire de 201 100 g/mole. La teneur en méthoxy polyéthylène glycol non réagi est égale à 20,5 % en poids du méthoxy initial, soit un rendement calculé de méthoxy effectivement greffé de 55,5 % en poids du méthoxy initial.

Ces résultats démontrent clairement que l'estérification directe à haute température d'un polyacide méthacrylique s'effectue avec des rendements élevés si l'on considère la disparition de la fonction acide. En revanche, la forte teneur en polyéthylène glycol libre démontre que les méthoxy ne sont pas stables pour ces températures, ce qui conduit à des dégradations thermiques produisant une libération des chaînes pendantes.

### Exemple 2

Cet exemple illustre l'invention, c'est-à-dire le procédé tel que décrit dans le document FR 2 900 930 et dans lequel on a mis en oeuvre un antioxydant.
Pour chacun des essais n° 3 à 9, on commence par prendre une solution d'un homopolymère de l'acide méthacrylique et à sécher celle-ci selon les étapes a) et b) du procédé de l'invention, avec un protocole identique à celui des essais 1 et 2.

### Essai n° 3

Selon l'étape c) du procédé de l'invention, dans un réacteur en verre de 0,25 litre muni d'une agitation mécanique et d'un chauffage électrique on mélange :
- 80 g de Polyglykol™ M 2000,
- 0,24 g d'hydroxyde de lithium,
- 13,8 g de l'homopolymère de l'acide méthacrylique sec obtenu par la méthode de séchage précédemment décrite (soit 0,16 mole d'acide méthacrylique),
- 0,20 g d'Irganox™ 5057 commercialisé par la société CIBA™.

Selon l'étape d) du procédé de l'invention, intervient alors la réaction d'estérification. L'ensemble est dégazé par un courant d'azote pendant 20 minutes. Puis sous agitation, on monte progressivement la température jusqu'à 190°C et on met l'ensemble sous vide (20 mm de mercure) cette montée en température s'effectuant en 60 minutes. On cuit alors le mélange durant 120 minutes à 190°C. L'ensemble est alors refroidi et dilué avec 128 g d'eau puis neutralisé avec de la soude 50 % jusqu'à pH 8,4.

### Essai n° 4

Selon l'étape c) du procédé de l'invention, dans un réacteur en verre de 0,25 litre muni d'une agitation mécanique et d'un chauffage électrique on mélange :
- 36,1 g de Polyglykol™ M 2000, sous forme fondu,
- 0,24 g d'hydroxyde de lithium,
- 15,68 g de l'homopolymère de l'acide méthacrylique sec obtenu par la méthode de séchage précédemment décrite,
- 0,18 g d'Irganox™ 5057.

Selon l'étape d) du procédé de l'invention, intervient alors la réaction d'estérification. L'ensemble est dégazé par un courant d'azote pendant 20 minutes. Puis sous agitation, on monte progressivement la température jusqu'à 210°C et on met l'ensemble sous vide (20 mm de mercure) cette montée en température s'effectuant en 60 minutes. On cuit alors le mélange durant 30 minutes à 210°C. L'ensemble est alors refroidi et dilué avec 78,3 g d'eau puis neutralisé avec de la soude 50 % jusqu'à pH 8,4.

### Essai n° 5

Selon l'étape c) du procédé de l'invention, dans un réacteur en verre de 0,25 litre muni d'une agitation mécanique et d'un chauffage électrique on mélange :
- 52,1 g de Polyglykol™ M 2000, sous forme fondu,
- 0,24 g d'hydroxyde de lithium,
- 15 g de l'homopolymère de l'acide méthacrylique sec obtenu par la méthode de séchage précédemment décrite,
- 0,2 g d'Irganox™ 5057.

Selon l'étape d) du procédé de l'invention, intervient alors la réaction d'estérification. L'ensemble est dégazé par un courant d'azote pendant 20 minutes. Puis sous agitation, on monte progressivement la température jusqu'à 210°C et on met l'ensemble sous vide (20 mm de mercure) cette montée en température s'effectuant en 45 minutes. On cuit alors le mélange durant 30 mn à 210°C. L'ensemble est alors refroidi et dilué avec 105 g d'eau puis neutralisé avec de la soude 50 % jusqu'à pH 8,4.

### Essai n° 6

Selon l'étape c) du procédé de l'invention, dans un réacteur en verre de 0,25 litre muni d'une agitation mécanique et d'un chauffage électrique on mélange :
- 44,1 g de Polyglykol™ M 2000, sous forme fondu,
- 0,24 g d'hydroxyde de lithium,
- 15,34 g de l'homopolymère de l'acide méthacrylique sec obtenu par la méthode de séchage précédemment décrite,
- 0,2 g d'Irganox™ 5057.

Selon l'étape d) du procédé de l'invention, intervient alors la réaction d'estérification. L'ensemble est dégazé par un courant d'azote pendant 20 minutes. Puis sous agitation, on monte progressivement la température jusqu'à 210°C et on met l'ensemble sous vide (20 mm de mercure) cette montée en température s'effectuant en 45 minutes. On cuit alors le mélange durant 60 minutes à 210°C. L'ensemble est alors refroidi et dilué avec 89,8 g d'eau puis neutralisé avec de la soude 50 % jusqu'à pH 8,4.

### Essai n° 7

Selon l'étape c) du procédé de l'invention, dans un réacteur en verre de 0,25 litre muni d'une agitation mécanique et d'un chauffage électrique on mélange :
- 80 g de Polyglykol™ M 2000, sous forme fondu,
- 1,9 g d'acide p-toluène sulfonique,
- 13,8 g de l'homopolymère de l'acide méthacrylique sec obtenu par la méthode de séchage précédemment décrite,
- 0,20 g d'Irganox™ 5057.

Selon l'étape d) du procédé de l'invention, intervient alors la réaction d'estérification. L'ensemble est dégazé par un courant d'azote pendant 20 minutes. Puis sous agitation, on monte progressivement la température jusqu'à 190°C et on met l'ensemble sous vide (20 mm de mercure) cette montée en température s'effectuant en 60 minutes. On cuit alors le mélange durant 120 minutes à 190°C. L'ensemble est alors refroidi et dilué avec 128 g d'eau puis neutralisé avec de la soude 50 % jusqu'à pH 8,4.

### Essai n° 8

Selon l'étape c) du procédé de l'invention, dans un réacteur en verre de 0,25 litre muni d'une agitation mécanique et d'un chauffage électrique on mélange :
- 80 g de Polyglykol™ M 2000, sous forme fondu,
- 0,24 g d'hydroxyde de lithium,
- 13,8 g de l'homopolymère de l'acide méthacrylique sec obtenu par la méthode de séchage précédemment décrite,
- 0,20 g de Doverphos™ 7 commercialisé par la société DOVER CHEMICAL™.

Selon l'étape d) du procédé de l'invention, intervient alors la réaction d'estérification. L'ensemble est dégazé par un courant d'azote pendant 20 minutes. Puis sous agitation, on monte progressivement la température jusqu'à 210°C et on met l'ensemble sous vide (20 mm de mercure) cette montée en température s'effectuant en 60 minutes. On cuit alors le mélange durant 30 minutes à 210°C. L'ensemble est alors refroidi et dilué avec 128 g d'eau puis neutralisé avec de la soude 50 % jusqu'à pH 8,4.

### Essai n° 9

Selon l'étape c) du procédé de l'invention, dans un réacteur en verre de 0,25 litre muni d'une agitation mécanique et d'un chauffage électrique on mélange :
- 80 g de Polyglykol™ M 2000 commercialisé par la société CLARIANT™, sous forme fondu,
- 0,24 g d'hydroxyde de lithium,
- 13,8 g de l'homopolymère de l'acide méthacrylique sec obtenu par la méthode de séchage précédemment décrite,
- 0,20 g de Doverphos™ 10 commercialisé par la société DOVER CHEMICAL™.

Selon l'étape d) du procédé de l'invention, intervient alors la réaction d'estérification. L'ensemble est dégazé par un courant d'azote pendant 20 minutes. Puis sous agitation, on monte progressivement la température jusqu'à 210°C et on met l'ensemble sous vide (20 mm de mercure) cette montée en température s'effectuant en 60 minutes. On cuit alors le mélange durant 30 minutes à 210°C. L'ensemble est alors refroidi et dilué avec 128 g d'eau puis neutralisé avec de la soude 50 % jusqu'à pH 8,4.

Les résultats obtenus figurent dans le tableau 1.

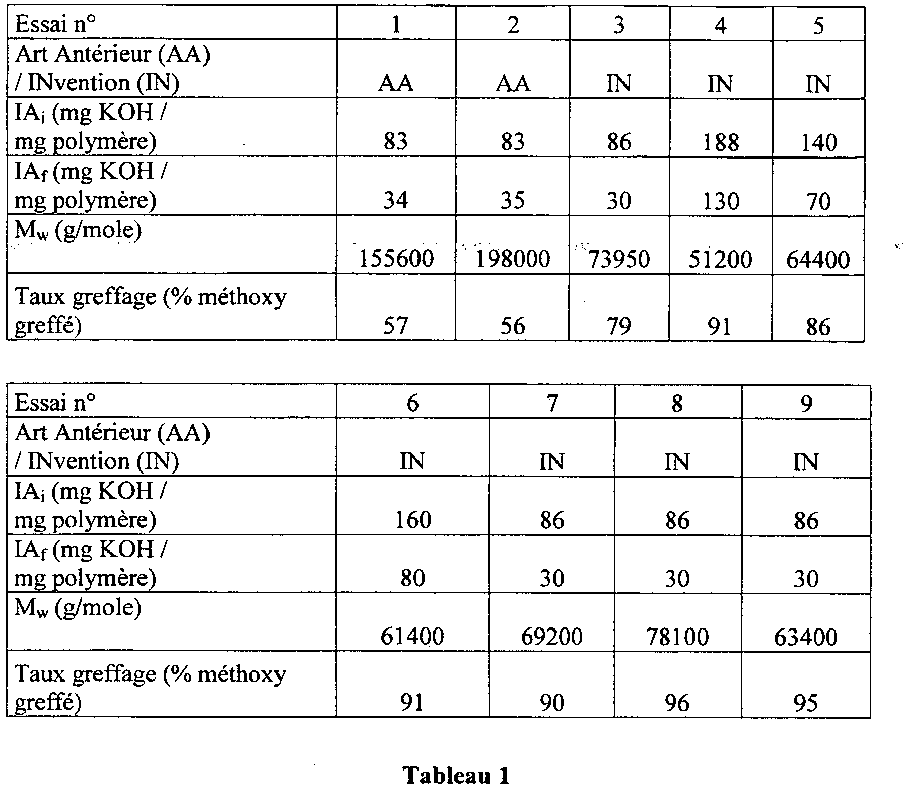

Ces résultats démontrent clairement que l'estérification directe à haute température d'un polyacide méthacrylique s'effectue avec des rendements élevés si on considère uniquement la disparition de la fonction acide. En revanche, la forte teneur en groupements polyoxyéthylène libres, de même que la masse moléculaire élevée du polymère final démontrent que les groupements méthoxy polyoxyéthylène ne sont pas stables aux températures où on fonctionnalise l'acide polyméthacrylique. Ceci conduit à une dégradation thermique du polymère peigne produisant une libération des chaînes polyoxyéthylène pendantes.

Par opposition, la mise en oeuvre d'un antioxydant permet, tout en conservant un degré de substitution identique au niveau des fonctions carboxyliques, d'obtenir un polymère greffé de plus bas poids moléculaire et avec une teneur en groupes polyoxyéthylène plus faible que dans les essais 1 et 2 : on a donc très largement amélioré le rendement du procédé de l'art antérieur.

## Revendications

1. Procédé de fabrication de polymères peignes disposant d'une chaîne principale (méth)acrylique et de groupements latéraux polyoxyalkylés, consistant à :
a) réaliser une solution contenant au moins un homopolymère et / ou copolymère de l'acide (méth)acrylique avec au moins un autre monomère,
b) sécher la solution obtenue à l'étape a), en vue d'obtenir un homopolymère et / ou un copolymère de l'acide (méth)acrylique avec au moins un autre monomère à l'état sec,
c) mélanger le produit à l'état sec obtenu selon l'étape b) avec au moins un alcoxy polyoxyalkylène glycol à l'état fondu et / ou au moins un alcoxy polyoxyalkylène amine à l'état fondu et / ou au moins un oxyde d'alkylène à l'état liquide ou gazeux,
d) fonctionnaliser alors l'homopolymère et / ou le copolymère de l'acide (méth)acrylique par :
- estérification avec au moins un alcoxy polyoxyalkylène glycol,
- et / ou amidification avec au moins un alcoxy polyoxyalkylène amine,
- ou éthoxylation avec au moins un oxyde d'alkylène.
et **caractérisé en ce qu'**on introduit un antioxydant pendant l'étape a) et /ou l'étape b) et / ou l'étape c).

2. Procédé selon la revendication 1, **caractérisé en ce que** l'antioxydant est une amine ayant au moins un groupement aromatique substitué par une chaîne alkyle, préférentiellement deux groupement aromatiques dont l'un au moins est substitué par une chaîne alkyle ayant de 3 à 9 atomes de carbone et est très préférentiellement le composé dont le numéro CAS est le 68411-46-1.

3. Procédé selon la revendication 1, **caractérisé en ce que** l'antioxydant est composé organophosphate, préférentiellement un phosphite aromatique et/ou aliphatique, et est très préférentiellement choisi parmi les composés dont les numéros CAS sont 25550-98-5 et 101-02-0.

4. Procédé selon l'une des revendications 1 à 3, **caractérisé en ce que** l'étape c) est effectuée à une température comprise entre 150°C et 250°C, préférentiellement entre 180°C et 220°C.

5. Procédé selon l'une des revendications 1 à 4, **caractérisé en ce que** l'étape c) est effectuée en présence d'un catalyseur, ledit catalyseur étant préférentiellement choisi parmi l'acide p-toluène sulfonique et l'hydroxyde de lithium.

6. Procédé selon l'une des revendications 1 à 5, **caractérisé en ce que** l'alcoxy polyoxyalkylène glycol est un méthoxy polyoxyalkylène glycol, et préférentiellement un méthoxy polyoxyéthylène glycol.

7. Procédé selon l'une des revendications 1 à 5, **caractérisé en ce que** l'alcoxy polyoxyalkylène amine est un méthoxy polyoxyalkylène amine, et préférentiellement un méthoxy polyoxyéthylène amine.

8. Procédé selon l'une des revendications 1 à 5, **caractérisé en ce que** l'oxyde d'alkylène est un oxyde d'éthylène, ou de propylène ou leurs mélanges.

9. Procédé selon l'une des revendications 1 à 8, **caractérisé en ce que** l'homopolymère et / ou le copolymère de l'acide (méth)acrylique est obtenu par des procédés de polymérisation radicalaire en solution, en émulsion directe, en présence de systèmes catalytiques et d'agents de transfert, ou encore par des procédés de polymérisation radicalaire contrôlée et préférentiellement par la polymérisation contrôlée par des nitroxydes (NMP) ou par des cobaltoximes, la polymérisation par transfert d'atome radicalaire (ATRP), la polymérisation radicalaire contrôlée par des dérivés soufrés, choisis parmi des carbamates, des dithioesters ou des trithiocarbonates (RAFT) ou des xanthates.

10. Procédé selon l'une des revendications 1 à 9, **caractérisé en ce que** l'autre monomère du copolymère de l'acide (méth)acrylique est choisi parmi :
- au moins un monomère anionique à insaturation éthylénique et à fonction monocarboxylique qui est préférentiellement l'acide acrylique ou méthacrylique ou leurs mélanges,
- au moins un monomère à insaturation éthylénique, choisi parmi au moins un monomère à insaturation éthylénique et fonction dicarboxylique et est préférentiellement choisi parmi l'acide crotonique, itaconique, maléique, ou encore les anhydrides d'acides carboxyliques, et est préférentiellement l'anhydride maléique ou choisi parmi les monomères à insaturation éthylénique et à fonction sulfonique et est préférentiellement choisi parmi l'acide 2-acrylamido-2-méthyl-propane-sulfonique, l'acide vinyl sulfonique, ou les sels de l'acide allyl éther sulfonate, l'acide styrène sulfonique ou bien encore choisi parmi les monomères à insaturation éthylénique et à fonction phosphorique et est préférentiellement choisi parmi l'acide vinyl phosphorique, le phosphate de méthacrylate d'éthylène glycol, le phosphate de méthacrylate de propylène glycol, le phosphate d'acrylate d'éthylène glycol, le phosphate d'acrylate de propylène glycol et leurs éthoxylats ou bien encore choisi parmi les monomères à insaturation éthylénique et à fonction phosphonique et est préférentiellement l'acide vinyl phosphonique, ou encore choisi parmi la vinylcaprolactone ou la vinylpyrrolidone, ou leurs mélanges,
- au moins un monomère non hydrosoluble tel que les acrylates ou les méthacrylates d'alkyles, ou leurs mélanges,
ou les mélanges de ces monomères.

11. Procédé selon l'une des revendications 1 à 10, **caractérisé en ce que** le séchage de l'homopolymère et / ou du copolymère de l'acide (méth)acrylique réalisé selon l'étape b) est effectué dans un sécheur par atomisation.

12. Procédé selon l'une des revendications 1 à 11, **caractérisé en ce que** le séchage de l'homopolymère et / ou du copolymère de l'acide (méth)acrylique réalisé selon l'étape b), est effectué jusqu'à obtenir un taux de matière sèche supérieur à 80 %, préférentiellement 90 %, et très préférentiellement supérieur à 95 % du poids dudit homopolymère et / ou dudit copolymère, tel que mesuré par une balance-dessicateur commercialisée par la société METTLER-TOLEDO™ sous le nom de HR83, la mesure étant réalisée par séchage à 150°C jusqu'à obtenir une perte en masse inférieure à 1 mg pendant 30 secondes.

13. Procédé selon l'une des revendications 1 à 12, **caractérisé en ce qu'**il comporte une étape éventuelle e), ultérieure à l'étape d), qui consiste à mettre en solution aqueuse le produit obtenu à l'issu de l'étape d), puis éventuellement à neutraliser totalement ou partiellement ledit produit par un ou plusieurs agents de neutralisation.

14. Procédé selon la revendication 13, **caractérisé en ce que** les agents de neutralisation sont choisis parmi ceux disposant d'une fonction neutralisante monovalente et sont alors préférentiellement choisis parmi les cations alcalins, et très préférentiellement choisis parmi le sodium, le potassium, le lithium, l'ammonium ou sont préférentiellement choisis parmi les amines primaires, secondaires, tertiaires aliphatiques et / ou cycliques, et très préférentiellement choisis parmi la stéarylamine, les éthanolamines (mono-, di-, triéthanolamine), la mono et diéthylamine, la cyclohexylamine, la méthylcyclohexylamine, l'amino méthyl propanol, la morpholine, ou sont choisis parmi les agents de neutralisation disposant d'une fonction neutralisante divalente et sont préférentiellement choisis parmi les cations divalents alcalino-terreux, et sont très préférentiellement choisis parmi le magnésium, le calcium, le zinc, ou sont choisis parmi les agents de neutralisation disposant d'une fonction neutralisante trivalente et consistent préférentiellement en l'aluminium, ou sont choisis parmi les agents de neutralisation disposant d'une fonction neutralisante plus élevée que la fonction trivalente, ou sont choisis parmi leurs mélanges.

15. Polymères peignes, obtenus par le procédé selon l'une des revendications 1 à 12, à l'état sec, disposant d'une chaîne principale (méth)acrylique et de groupements latéraux polyoxyalkylés greffés, **caractérisés en ce qu'**ils présentent un taux de greffage supérieur à 70 %, préférentiellement 80 %, très préférentiellement 90 %, tel que mesuré par GPC.

16. Polymères peigne, à l'état sec, selon la revendication 15, **caractérisés en ce qu'**ils présentent un taux de matière sèche supérieur à 80 %, préférentiellement supérieur à 85 %, très préférentiellement supérieur à 90 %, et extrêmement préférentiellement supérieur à 95 % de leur poids, tel que mesuré par une balance-dessicateur commercialisée par la société METTLER-TOLEDO™ sous le nom de HR83, la mesure étant réalisée par séchage à 150°C jusqu'à obtenir une perte en masse inférieure à 1 mg pendant 30 secondes.

17. Polymères peignes, obtenus par le procédé selon l'une des revendications 1 à 14, en solution aqueuse, disposant d'une chaîne principale (méth)acrylique et de groupements latéraux polyoxyalkylés greffés, **caractérisés en ce qu'**ils présentent un taux de greffage supérieur à 70 %, préférentiellement 80 %, très préférentiellement 90 %, tel que mesuré par GPC.

18. Formulations contenant des matières minérales ou organiques, **caractérisées en ce qu'**elles contiennent au moins un polymère peigne selon l'une des revendications 15 à 17.

19. Formulations selon la revendication 18, **caractérisées en ce qu'**elles sont des formulations aqueuses contenant des matières minérales ou organiques, lesdites formulations étant choisies parmi des dispersions ou des suspensions aqueuses de matières minérales ou organiques, contenant éventuellement un agent de dispersion et / ou un agent d'aide au broyage et / ou un agent anti sédimentation et / ou un agent épaississant, ou des compositions aqueuses à base de liants hydrauliques, et sont préférentiellement des bétons, des mortiers, des laitiers, des coulis, des plâtres, ou des sauce de couchage papetière, ou des peintures, ou des formulations cosmétiques ou détergentes, ou des formulations textiles, ou des formulations céramiques, ou des boues de forage.

## Patentansprüche

1. Verfahren zur Herstellung von Kammpolymeren mit einer (Meth)acrylsäure-Hauptkette und polyalkoxylierten Seitengruppen, wobei es darin besteht :
a) eine Lösung herzustellen, die mindestens ein Homopolymer und/oder Copolymer von (Meth)acrylsäure mit mindestens einem weiteren Monomer enthält,
b) die Lösung, welche in Schritt a) erhalten wurde, zu trocknen, um ein Homopolymer und/oder Copolymer von (Meth)acrylsäure mit mindestens einem weiteren Monomer in trockenem Zustand zu erhalten,
c) das Produkt in trockenem Zustand, das in Schritt b) erhalten wurde, mit mindestens einem Alkoxy-Polyalkylenoxid-Glykol in geschmolzenem Zustand und/oder mindestens einem Alkoxy-Polyalkylenoxid-Amin in geschmolzenem Zustand und/oder mindestens einem Alkylenoxid in flüssigem oder gasförmigem Zustand zu vermischen,
d) das Homopolymer und/oder Copolymer von (Meth)acrylsäure daraufhin folgendermaßen mit funktionellen Gruppe zu versehen:
- durch Veresterung mit mindestens einem Alkoxy-Polyalkylenoxid-Glykol,
- und/oder durch Amidierung mit mindestens einem Alkoxy-Polyalkylenoxid-Amin,
- oder Ethoxylierung mit mindestens einem Alkylenoxid,
und es **dadurch gekennzeichnet ist, dass** während des Schrittes a) und/oder des Schrittes b) und/oder des Schrittes c) ein Antioxidans zugesetzt wird.

2. Verfahren nach Anspruch 1, **dadurch gekennzeichnet, dass** es sich bei dem Antioxidans um ein Amin handelt, das mindestens eine aromatische Gruppe aufweist, welche mit einer Alkylkette substituiert ist, vorzugsweise zwei aromatische Gruppen, von welchen mindestens eine mit einer Alkylkette mit 3 bis 9 Kohlenstoffatomen substituiert ist, und es sich stark bevorzugt um die Verbindung mit der CAS-Nummer 68411-46-1 handelt.

3. Verfahren nach Anspruch 1, **dadurch gekennzeichnet, dass** es sich bei dem Antioxidans um eine Organophosphorverbindung handelt, vorzugsweise um ein aromatisches und/oder aliphatisches Phosphit, wobei sie stark bevorzugt aus den Verbindungen mit den CAS-Nummern 25550-98-5 und 101-02-0 ausgewählt ist.

4. Verfahren nach einem der Ansprüche 1 bis 3, **dadurch gekennzeichnet, dass** der Schritt c) bei einer Temperatur im Bereich von 150°C bis 250°C, vorzugsweise von 180°C bis 220°C, durchgeführt wird.

5. Verfahren nach einem der Ansprüche 1 bis 4, **dadurch gekennzeichnet, dass** der Schritt c) in Gegenwart eines Katalysators durchgeführt wird, wobei der Katalysator vorzugsweise aus p-Toluolsulfonsäure und Lithiumhydroxid ausgewählt ist.

6. Verfahren nach einem der Ansprüche 1 bis 5, **dadurch gekennzeichnet, dass** es sich bei dem Alkoxy-Polyalkylenoxid-Glykol um einen Methoxy-Polyalkylenoxid-Glykol und vorzugsweise um einen Methoxy-Polyethylenoxid-Glykol handelt.

7. Verfahren nach einem der Ansprüche 1 bis 5, **dadurch gekennzeichnet, dass** es sich bei dem Alkoxy-Polyalkylenoxid-Amin um ein Methoxy-Polyalkylenoxid-Amin und vorzugsweise um einen Methoxy-Polyethylenoxid-Amin handelt.

8. Verfahren nach einem der Ansprüche 1 bis 5, **dadurch gekennzeichnet, dass** es sich bei dem Alkylenoxid um ein Ethylen- oder Propylenoxid oder um deren Mischungen handelt.

9. Verfahren nach einem der Ansprüche 1 bis 8, **dadurch gekennzeichnet, dass** das Homopolymer und/oder Copolymer von (Meth)acrylsäure durch Verfahren der radikalischen Polymerisation in Lösung, in direkter Emulsion, in Gegenwart von katalytischen Systemen und Kettenübertragungsmitteln erhalten wird, oder auch durch Verfahren der kontrollierten radikalischen Polymerisation und vorzugsweise durch eine Polymerisation, welche mittels Nitroxiden (NMP) oder mittels Cobaloximen kontrolliert wird, durch eine radikalische Polymerisation unter Atomtransfer (ATRP), durch eine radikalische Polymerisation, welche mittels schwefelhaltiger Verbindungen, die aus den Carbamaten, den Dithioestern oder den Trithiocarbonaten (RAFT) oder den Xanthogenaten ausgewählt sind, kontrolliert wird.

10. Verfahren nach einem der Ansprüche 1 bis 9, **dadurch gekennzeichnet, dass** das weitere Monomer des Copolymers von (Meth)acrylsäure aus den folgenden ausgewählt ist:
- mindestens einem ethylenisch ungesättigten anionischen Monomer mit funktioneller Monocarbonsäuregruppe, bei welchem es sich vorzugsweise Acryl- oder Methacrylsäure oder deren Mischungen handelt,
- mindestens einem ethylenisch ungesättigten Monomer, das aus mindestens einem ethylenisch ungesättigten Monomer mit funktioneller Dicarbonsäuregruppe ausgewählt ist, wobei es vorzugsweise aus Crotonsäure, Itaconsäure, Maleinsäure ausgewählt ist, oder auch aus den Carbonsäureanhydriden ausgewählt ist, wobei es sich vorzugsweise um Maleinsäureanhydrid handelt, oder aus den ethylenisch ungesättigten Monomeren mit funktioneller Sulfonsäuregruppe ausgewählt ist, wobei es vorzugsweise aus 2-Acrylamido-2-methylpropansulfonsäure, Vinylsulfonsäure, oder den Salzen des Typs Allylethersulfonat, Styrolsulfonsäure ausgewählt ist, oder auch aus den ethylenisch ungesättigten Monomeren mit funktioneller Phosphorsäuregruppe ausgewählt ist, wobei es vorzugsweise aus Vinylphosphorsäure, Ethylenglykolmethacrylatphosphat, Propylenglykolmethacrylatphosphat, Ethylenglykolacrylatphosphat, Propylenglykolacrylatphosphat und deren Ethoxylaten ausgewählt ist, oder auch aus den ethylenisch ungesättigten Monomeren mit funktioneller Phosphonsäure ausgewählt ist, wobei es sich vorzugsweise um Vinylphosphonsäure handelt, oder auch aus Vinylcaprolacton oder Vinylpyrrolidon oder deren Mischungen ausgewählt ist,
- mindestens einem wasserunlöslichen Monomer wie etwa den Alkylacrylaten oder methacrylaten oder deren Mischungen,
oder den Mischungen dieser Monomere.

11. Verfahren nach einem der Ansprüche 1 bis 10, **dadurch gekennzeichnet, dass** das Trocknen des Homopolymers und/oder des Copolymers von (Meth)acrylsäure, welches gemäß Schritt b) erfolgt, in einer Sprühtrocknungsvorrichtung durchgeführt wird.

12. Verfahren nach einem der Ansprüche 1 bis 11, **dadurch gekennzeichnet, dass** das Trocknen des Homopolymers und/oder des Copolymers von (Meth)acrylsäure, welches gemäß Schritt b) erfolgt, solange durchgeführt wird, bis ein Trockenmassegehalt von mehr als 80 %, vorzugsweise 90 %, und stark bevorzugt mehr als 95 % erreicht ist, bezogen auf das Gewicht des Homopolymers und/oder des Copolymer, gemäß einer Messung mittels eines gravimetrischen Feuchtigkeitsmessgeräts, welches von der Firma METTLER-TOLEDO^{™} unter der Bezeichnung HR83 vertrieben wird, wobei die Messung erfolgt, indem bei 150°C solange getrocknet wird, bis der Massenverlust über einen Zeitraum von 30 Sekunden weniger als 1 mg beträgt.

13. Verfahren nach einem der Ansprüche 1 bis 12, **dadurch gekennzeichnet, dass** es nach dem Schritt d) möglicherweise einen Schritt e) aufweist, der darin besteht, das Produkt, welches nach Abschluss von Schritt d) erhalten wird, in Wasser aufzulösen und das Produkt anschließend möglicherweise mit einem oder mehreren Neutralisierungsmitteln teilweise oder vollständig zu neutralisieren.

14. Verfahren nach Anspruch 13, **dadurch gekennzeichnet, dass** die Neutralisierungsmittel aus den denjenigen ausgewählt sind, die eine einwertige neutralisierende funktionelle Gruppe aufweisen, wobei sie in diesem Falle vorzugsweise aus den Alkalikationen und zwar stark bevorzugt aus Natrium, Kalium, Lithium, Ammonium ausgewählt sind, oder vorzugweise aus den aliphatischen und/oder zyklischen primären, sekundären, tertiären Aminen, und zwar stark bevorzugt aus Stearylamin, den Ethanolaminen (Mono-, Di-, Triethanolamin), Mono- und Diethylamin, Cyclohexylamin, Methylcyclohexylamin, Aminomethylpropanol, Morpholin ausgewählt sind, oder sie aus den Neutralisierungsmitteln ausgewählt sind, die eine zweiwertige neutralisierende funktionelle Gruppe aufweisen, wobei sie vorzugweise aus den zweiwertigen Erdalkalikationen und zwar stark bevorzugt aus Magnesium, Calcium, Zink ausgewählt sind, oder sie aus den Neutralisierungsmitteln ausgewählt sind, die eine dreiwertige neutralisierende funktionelle Gruppe aufweisen, wobei es sich vorzugsweise um Aluminium handelt, oder sie aus den Neutralisierungsmitteln ausgewählt sind, die eine neutralisierende funktionelle Gruppe aufweisen, die mehr als dreiwertig ist, oder sie aus deren Mischungen ausgewählt sind.

15. Kammpolymere, die mittels des Verfahrens nach einem der Ansprüche 1 bis 12 in trockenem Zustand erhalten wurden, wobei sie eine (Meth)acrylsäure-Hauptkette und gepfropfte polyalkoxylierte Seitengruppen aufweisen und **dadurch gekennzeichnet sind, dass** ihr Pfropfungsgrad mehr als 70 %, vorzugsweise 80 %, stark bevorzugt 90 % beträgt, wobei dieser mittels GPC gemessen wird.

16. Kammpolymere in trockenem Zustand nach Anspruch 15, **dadurch gekennzeichnet, dass** sie eine Trockenmassegehalt von mehr als 80 %, vorzugsweise von mehr als 85 %, stark bevorzugt von mehr als 90 %, und mit äußerstem Vorzug von mehr als 95 % ihres Gewichts aufweisen, gemäß einer Messung mittels eines gravimetrischen Feuchtigkeitsmessgeräts, welches von der Firma METTLER-TOLEDO^{™} unter der Bezeichnung HR83 vertrieben wird, wobei die Messung erfolgt, indem bei 150°C solange getrocknet wird, bis der Massenverlust über einen Zeitraum von 30 Sekunden weniger als 1 mg beträgt.

17. Kammpolymere, die mittels des Verfahrens nach einem der Ansprüche 1 bis 14 erhalten wurden, in wässriger Lösung, wobei sie eine (Meth)acrylsäure-Hauptkette und gepfropfte polyalkoxylierte Seitengruppen aufweisen und **dadurch gekennzeichnet sind, dass** ihr Pfropfungsgrad mehr als 70 %, vorzugsweise 80 %, stark bevorzugt 90 % beträgt, wobei dieser mittels GPC gemessen wird.

18. Formulierungen, die mineralische oder organische Stoffe enthalten, **dadurch gekennzeichnet, dass** sie mindestens ein Kammpolymer nach einem der Ansprüche 15 bis 17 enthalten.

19. Formulierungen nach Anspruch 18, **dadurch gekennzeichnet, dass** es sich um wässrige Formulierungen handelt, die mineralische oder organische Stoffe enthalten, wobei die Formulierungen aus den Dispersionen oder wässrigen Suspensionen mineralischer oder organischer Stoffe, die möglicherweise ein Dispersionsmittel und/oder ein Mahlhilfsmittel und/oder ein Antiabsetzmittel und/oder ein Verdickungsmittel enthalten, oder den wässrigen Zusammensetzungen auf Basis hydraulischer Bindemittel, bei welchen es sich vorzugsweise um Betonsorten, Mörtelsorten, Schlacken, Schlämmen, Gipse handelt, oder den Papierstreichmassen, oder den Anstrichmitteln, oder den kosmetischen oder reinigenden Formulierungen, oder den Textilformulierungen, oder den Keramikformulierungen, oder den Bohrschlämmen ausgewählt sind.

## Claims

1. A method of manufacturing comb polymers having a main (meth)acrylic chain and polyoxyalkylated lateral groups, consisting of:
a) producing a solution containing at least one homopolymer and/or copolymer of (meth)acrylic acid with at least one other monomer,
b) drying the solution obtained in step a), in view of obtaining a homopolymer and/or a copolymer of (meth)acrylic acid with at least one other monomer in a dry state,
c) mixing the product in a dry state obtained according to step b) with at least one alkoxy polyoxyalkylene glycol in a molten state, and/or at least one alkoxy polyoxyalkylene amine in a molten state, and/or at least one alkylene oxide in a liquid or gaseous state,
d) functionalizing the homopolymer and/or copolymer of (meth)acrylic acid by:
- esterification with at least one alkoxy polyoxyalkylene glycol,
- and/or amidification with at least one alkoxy polyoxyalkylene amine,
- or ethoxylation with at least one alkylene oxide,
and **characterized in that** an antioxidant is added during step a) and/or step b) and/or step c).

2. A method according to claim 1, **characterized in that** the antioxidant is an amine having at least one aromatic group substituted by an alkyl chain, preferentially two aromatic groups, at least one of which is substituted by an alkyl chain having 3 to 9 carbon atoms and is very preferentially the compound whose CAS number is 68411-46-1.

3. A method according to claim 1, **characterized in that** the antioxidant is an organophosphate compound, preferentially an aromatic and/or aliphatic phosphite, and is very preferentially chosen from among the compounds whose CAS numbers are 25550-98-5 and 101-02-0.

4. A method according to one of the claims 1 to 3, **characterized in that** step c) is carried out at a temperature between 150°C and 250°C, preferentially between 180°C and 220°C.

5. A method according to one of the claims 1 to 4, **characterized in that** step c) is carried out in the presence of a catalyst, said catalyst being preferentially chosen between p-toluenesulfonic acid and lithium hydroxide.

6. A method according to one of the claims 1 to 5, **characterized in that** the alkoxy polyoxyalkylene glycol is a methoxy polyoxyalkylene glycol, and preferentially a methoxy polyoxyethylene glycol.

7. A method according to one of the claims 1 to 5, **characterized in that** the alkoxy polyoxyalkylene amine is a methoxy polyoxyalkylene amine, and preferentially a methoxy polyoxyethylene amine.

8. A method according to one of the claims 1 to 5, **characterized in that** the alkylene oxide is an ethylene oxide, or a propylene oxide, or mixtures thereof.

9. A method according to one of the claims 1 to 8, **characterized in that** the homopolymer and/or copolymer of (meth)acrylic acid is obtained by methods of radical polymerization in solution, in a direct emulsion, in the presence of catalytic systems and transfer agents, or by processes of controlled radical polymerization, and preferentially by controlled polymerization by nitroxides (NMP) or by cobalt-oximes, by atom transfer radical polymerization (ATRP), controlled radical polymerization by sulphurated derivatives, said derivatives are chosen from among carbamates, dithioesters or trithiocarbonates (RAFT) or xanthates.

10. A method according to one of the claims 1 to 9, **characterized in that** the other monomer of the copolymer of (meth)acrylic acid is chosen from among:
- at least one ethylene-unsaturated anionic monomer with a monocarboxylic function which is preferentially acrylic or methacrylic acid, or mixtures thereof,
- at least one ethylenically-unsaturated monomer, chosen from among at least one ethylenically-unsaturated monomer with a dicarboxylic function, which is preferentially chosen from among crotonic, itaconic, or maleic acid, or from carboxylic-acid anhydrides, and is preferentially maleic anhydride or is chosen from among ethylenically-unsaturated monomers with a sulfonic function and is preferentially chosen from among 2-acrylamido-2-methyl-propane-sulfonic acid, vinylsulfonic acid, or salts of the allyl ether sulfonate acid, the styrene sulfonic acid, or chosen from among ethylenically-unsaturated monomers with a phosphoric function, and is preferentially chosen from among vinylphosphoric acid, ethylene glycol methacrylate phosphate, propylene glycol methacrylate phosphate, ethylene glycol acrylate phosphate, propylene glycol acrylate phosphate, and their ethoxylates, or from among ethylenically-unsaturated monomers with a phosphonic function, and is preferentially vinylphosphonic acid, or chosen from among vinylcaprolactone or vinylpyrrolidone, or mixtures thereof,
- at least one water-insoluble monomer, such as alkyl acrylates or methacrylates, or mixtures thereof,
or mixtures of these monomers.

11. A method according to one of the claims 1 to 10, **characterized in that** the drying of the homopolymer and/or copolymer of (meth)acrylic acid carried out according to step b) is performed in a dryer by atomization.

12. A method according to one of the claims 1 to 11, **characterized in that** the drying of the homopolymer and/or copolymer of (meth)acrylic acid performed according to step b) is carried out until a solids content level greater than 80% is achieved, preferentially 90%, and very preferentially greater than 95% of the weight of said homopolymer and/or said copolymer, as measured by a halogen moisture analyzer sold by the company METTLER-TOLEDO™ under the name HR83, the measurement being carried out by drying at 150°C until a loss of mass less than 1 mg is achieved for 30 seconds.

13. A method according to one of the claims 1 to 12, **characterized in that** it comprises a potential step e) occurring after step d), which consists of placing the product obtained at the end of step d) into an aqueous solution, then potentially fully or partially neutralizing said product using one or more neutralization agents.

14. A method according to claim 13, further **characterized in that** the neutralization agents are chosen from among those with a monovalent neutralizing function and are preferentially chosen from among alkaline cations, and very preferentially chosen from among sodium, potassium, lithium, and ammonium, or are preferentially chosen from among primary, secondary, or tertiary aliphatic and/or cyclic amines, and very preferentially chosen from among stearylamine, ethanolamines (mono-, ditriethanolamine), mono- and diethylamine, cyclohexylamine, methylcyclohexylamine, amino-methyl propanol, and morpholine, or are chosen from among neutralization agents having a divalent neutralizing function, and are preferentially chosen from among divalent alkaline-earth cations, and are very preferentially chosen from among magnesium, calcium, and zinc, or are chosen from among neutralization agents having a trivalent neutralizing function and consist preferentially of aluminum, or are chosen from among neutralization agents having a more than trivalent neutralizing function, or are chosen from among mixtures thereof.

15. Comb polymers, obtained by a method according to one of the claims 1 to 12, in a dry state, having a main (meth)acrylic chain and grafted polyoxyalkylated lateral groups, **characterized in that** they exhibit a grafting rate greater than 70%, preferentially 80%, and very preferentially 90%, as measured by GPC.

16. Comb polymers, in a dry state, according to claim 15, **characterized in that** they exhibit a dry solids rate greater than 80%, preferentially greater than 85%, and very preferentially greater than 90%, and extremely preferentially greater than 95% of their weight, as measured by a halogen moisture analyzer sold by the company METTLER-TOLEDO™ under the name HR83, the measurement being carried out by drying at 150°C until a loss of mass less than 1 mg is obtained for 30 seconds.

17. Comb polymers, obtained by a method according to one of the claims 1 to 14, in an aqueous solution, having a main (meth)acrylic chain and grafted polyoxyalkylated lateral groups, **characterized in that** they exhibit a grafting rate greater than 70%, preferentially 80%, and very preferentially 90%, as measured by GPC.

18. Formulations containing mineral or organic materials, **characterized in that** they contain at least one comb polymer according to one of the claims 15 to 17.

19. Formulations according to claim 18, **characterized in that** they are aqueous formulations containing mineral or organic materials, said formulations being chosen from among aqueous dispersions or suspensions of mineral or organic materials, potentially containing a dispersion agent and/or a grinding aid agent and/or an anti-sedimentation agent and/or a thickening agent, or aqueous compositions based on hydraulic binders, and are preferentially concretes, mortars, slags, grouts, plasters, or paper coating colors, or paints, or cosmetic or detergent formulations, or textile formulations, or ceramic formulations, or drilling muds.
